# EUROPEAN PATENT APPLICATION

(11) **EP 1 570 875 A1**
(43) Date of publication of application: **07.09.2005**
(21) Application number: 04005086.6
(22) Date of filing: 04.03.2004
(51) Int. Cl.: A61M 5/20, A61M 5/30

(54) **Pyrotechnically driven needle-free liquid jet injection device with low-cost components and production**

(71) Applicant: Roche Diagnostics GmbH, 68305 Mannheim (DE); F. HOFFMANN-LA ROCHE AG, 4070 Basel (CH)
(72) Inventor: Arnitz, Theo, 76275 Ettlingen (DE); Fürst, Otto, 68519 Viernheim (DE); Haar, Hans-Peter, 69168 Wiesloch (DE); List, Hans, 64754 Hesseneck (DE); Meacham, George Bevan Kirby, Shaker Heights Ohio 44122 (US)
(74) Representative: Jany, Peter

(57) **Abstract**

An easy, precise and low-cost manufacturing of a pyrotechnically driven needle-free liquid jet injection device for performing a needleless hypodermic injection of a liquid medication contained in the device into a body, said device comprising a driving unit including pyrotechnical means for generating within the device a pressure necessary for injecting the medication, said means comprising a gas generating pyrotechnical material and an actuation device for igniting the pyrotechnical material, is achieved by said injection device comprising a one-piece ductile pressure shell surrounding at least a portion of the pyrotechnical material, said pressure shell being formed by a single deep-drawn material, preferably metal, as basic construction material for the shell.

## Description

Broadly, needle-free injection (NFI) (also called needleless hypodermic injection) includes any process that accelerates medication to high velocity so that it penetrates the skin and underlying tissue by its own momentum, thereby eliminating the need for a penetrating needle. In the needle-free injection field the primary NFI advantage is that without a needle, disease transmission through needle-stick injuries or needle reuse is eliminated. Other advantages include improved self medication and patient comfort, particularly for those with needle phobia.

The medications may be in the form of liquids or solid particles. The liquid in a NFI jet is typically pressurized to 200 to 300 bar and accelerated as a coherent stream by a jet nozzle with typically 0.10 to 0.30 millimeters diameter. The fluid stream with the medication forms one or more holes in the skin and tissue, and the liquid is deposited in the tissue. It is functionally similar to conventional needle injections, and allows delivery of a relatively large volume of material deep under the skin with minimal trauma. Further, the liquid medication formulation may be similar or identical to that currently supplied for needle injectors.

In solid particle NFI the particles are accelerated as a particle beam by a moving solid structure or entrainment in a high velocity gas stream. Each particle forms a separate hole in the skin and penetrates a depth dependent on its mass and velocity. In theory, large particles could penetrate deeply and deliver large medication doses, but the skin damage would be excessive. Solid particle NFI is therefore functionally different from conventional needle injections, and is best suited to medications such as vaccines where the dose mass is low and dermal rather than subcutaneous injection is required. It is also limited to medications that form stable and robust solid particles that disperse effectively in body tissue without causing adverse reactions such as inflammation. Liquid jet NFI has broader applications and greater commercial potential than solid particle NFI, and solid particle NFI will not be discussed further.

Liquid jet NFI has been described in patents and medical journals since around 1940. Many small systems have been developed for use by medical professionals and individual patents, and large systems have been developed to carry out mass inoculations. Mass inoculation systems typically draw measured doses from multidose ampoules, and use high pressure compressed gas as a power source. The value of jet injection in this application is that it facilitates automated injection. In early implementations the operator simply pressed the injection nozzle against the skin of each recipient, actuated the injector, and moved on to the next recipient. It was quicker and easier than loading and administering an individual hypodermic needle injection to each recipient, and was considered safe since there was no needle to transmit blood-born infections.

More recently, infection transmission through body fluid transfer from one recipient to the next on the surface of the injection nozzle has been identified. Such transmission is much less likely than with non-sterile needle reuse, but nonetheless has led to a requirement for disinfecting or replacing nozzles between injections. These extra steps eliminate some of the advantages of such systems.

Small systems, the principal subject of the present application, are typically self-contained hand-held devices that typically contain a single 0.1 to 1.0 milliliter medication dose and a power source that delivers 5 to 10 joules of energy in 10 to 200 milliseconds to pressurize the medication and to form the fluid jet. Reusable devices, disposable single use devices, and reusable devices with disposable cartridges have been developed for self-injection and clinical use. These employ a variety of medication handling methods and power sources.

Medication handling falls into two broad categories. In the first, the user transfers the liquid medication from the manufacturer's packaging to the NFI device. This is a technical operation that must be done with some care to preserve sterility, and may be difficult in cases in which patients self-inject or are injected by family members. In the second category, the medication is factory packaged in a sterile cartridge. This greatly simplifies the injection process and makes it accessible to a larger group of patients, but requires significant investment in package development, regulatory approval and production facilities.

Liquid jet NFI power sources include compressed gas, liquid gas, pyrotechnic gas generators and cocked springs. Compressed gas and pyrotechnics are depleted with each injection, and must be replaced regardless of whether the entire device is reusable. Springs may be recocked in reusable devices, although this requires a level of physical effort that may be difficult for some users. Viable small NFI systems have been developed with all of these approaches. Compressed gas and spring systems are the most common approaches, since they are relatively easy to develop and face less regulatory hurtles than pyrotechnic approaches. Pyrotechnic power sources, however, have a number of characteristics including small size, high performance, long shelf life and low cost that make them very attractive for mass production products.

This application focuses on systems combining sterile medication packages with pyrotechnic power sources. More narrowly, it focuses on small and low-cost systems that are discarded after a single use.

There is extensive known prior art in the small liquid jet NFI field, and much of that is described in the patent and medical literature. Examples of commercially available systems or systems in advanced development stages are briefly described in the following paragraphs.

The Bioject product described in US patent 5,399,163 is a commercially available system including a reusable power unit and single use disposable sterile injectors consisting of a piston and a polycarbonate cylinder and jet orifice. The power unit costs over $ 1000, and the injectors are about $ 1 each. The injectors are filled by the user from medication bottles closed by a rubber septum using a hollow needle adapter supplied with the injector. The adapter is attached to the injector, the needle is pushed through the septum and the plunger is manually withdrawn to draw in a medication dose. The adapter is removed from the injector and discarded, and the injector is then attached to the power unit. A replaceable CO₂ cartridge installed in the power unit supplies the energy for several injections.

A system of valves controls the CO₂ flow, and causes a large piston to be pressurized when the release button is pressed by the operator. This piston pushes the smaller piston in the injector to pressurize the medication and carry out the injection. The area ratio of the large piston in the power unit and the smaller piston in the injector boosts the maximum CO₂ pressure of about 50 bar to the required injection pressure of about 300 bar. The Bioject has excellent performance, but the cost of the power unit and the complexity of operation limit it to clinical use.

Use could be simplified if injectors were supplied filled with medication. This is an expensive development, however, since separate testing and regulatory approval are required for each medication, and may be beyond the means of smaller companies. Further, polycarbonate is not compatible with most medications for long-term storage contact, so injectors made of compatible materials must be developed.

The MediJect product described in US patent 5,919,159 is a commercially available insulin injection system that includes a reusable power unit and disposable sterile injectors consisting of a piston and a plastic cylinder and jet orifice. The power unit costs over $ 300, and the injectors are about $ 7 each. The injectors are intended for multiple injections over a 3 weeks period by a single diabetic patient. The injectors are filled by the user from insulin bottles closed by a rubber septum using a hollow needle adapter supplied with the injector. The adapter is attached to the injector installed in the power unit, the needle is pushed through the septum and the plunger is withdrawn to draw in the required insulin dose. The dose size is varied to match the patient's immediate needs.

The adapter is removed from the injector and saved for reuse. A spring and plunger in the power unit is cocked before each use to supply the injection energy. The release button is pressed by the operator to release the plunger and make the injection. The cocked spring accelerates the plunger, which impacts and then pushes the injector piston to pressurize the medication and carry out the injection. The impact generates an initial pressure spike of over 300 bar, followed by a pressure less than 300 bar for the balance of the injection.

The product has good performance, and the complexity of operation should not be a barrier for most diabetic patients who are accustomed to measuring and injecting insulin with hypodermic needles. Nonetheless, it has not penetrated the diabetes care market to a significant extent. This may be a combined result of cost, the effort required to cock the spring, and the needle injection expertise of typical diabetic patients. MediJect faces the same barriers as Bioject to supplying injectors filled with medications other than insulin. Insulin adds the complication of requiring variable dose size, with the result that excess medication is discarded.

The J-Tip product described in US patent 4,913,699 is a commercially available single use disposable injection device. It is targeted at insulin injection by diabetics, but may be used with other medications. Cost is about $ 2.50 each. The injectors are filled by the user from medication bottles closed by a rubber septum using a hollow needle syringe-like transfer device supplied with the injector. A CO₂ capsule in the device supplies the injection energy, and it is opened to pressurize a large piston just prior to use. The large piston is restrained by a latch until it is released by the operator to make the injection. This piston pushes the smaller piston in the injector to pressurize the medication and carry out the injection. The area ratio of the large piston in the power unit and the smaller piston in the injector boosts the maximum CO₂ pressure of about 50 bar to a higher pressure sufficient to make the injection.

While gas leakage and resulting low pressure and incomplete injections have reportedly been a problem, the J-Tip is an adequate injector. Further, the complexity of operation should not be a barrier for most diabetic patients who are accustomed to measuring and injecting insulin with hypodermic needles. Nonetheless, it has not penetrated the diabetes care market to a significant extent. This may be a result of cost, since diabetics typically make more than one injection per day. The needle injection expertise of typical diabetic patients may also be a factor. J-Tip faces the same barriers as MediJect and Bioject to supplying injectors filled with medication.

The Weston Intraject described in US patent 6,135,979 is a pre-commercial single use disposable injection device designed to be supplied filled with medication. The medication is contained in a glass cylinder that includes an integral jet orifice and is closed at the rear by a polymer piston. The nozzle opening is closed and kept sterile by a polymer plug held in place by a break-off portion of an outer shell surrounding the glass cylinder. This material combination is selected to allow long term storage of many different medications. The power module is a gas spring and ram held in a cocked position by a trigger latch.

The medication injector and the power unit are joined and shipped as an assembly. The injector is ready to use after breaking off a protective cap that guaranties sterility and removing a safety band that prevents actuation. It is then pressed against the injection site, thereby sliding a sleeve and releasing the trigger latch. This releases the gas spring and ram which impact and drive the polymer medication piston to make the injection. While the Intraject has generally good performance and is easy to use, reliability problems including gas leakage and glass breakage have reportedly prevented product commercialization.

The Roche RecoJet device described in US patent application publication US-2002/0169412-A1 is a pre-commercial system consisting of a reusable application device that accepts disposable cartridges containing medication and electrically ignited pyrotechnic propellant. It has a fixed injection volume, and is intended for medications such as erythropoietin. The application device includes a steel pressure shell that surrounds the cartridge, a battery for electric ignition, and an actuation button and safety interlock systems.

The cartridge contains the pyrotechnic gas generator and a flexible polyethylene medication reservoir that also encloses a polypropylene jet orifice. These materials have been proven to be compatible with erythropoietin for long term storage, and are expected to be similarly compatible with a number of other injectable medications. To prepare for use, the cartridge is loaded into the application device, the application device is locked closed, and the cover is removed to expose the sterile injection nozzle.
The nozzle is then pressed against the skin, in the process releasing a safety interlock. Finally, the actuation button is pressed to ignite the propellant. The injection is made as the gas from the generator pressurizes the exterior of the flexible medication reservoir and displaces the medication through the jet orifice. Afterwards, the used cartridge is removed and discarded.

Interchangeable spacer rings in the application device change the effective expansion volume of the gas generator and thereby adjust the injection pressure. Unlike stored CO₂ that has a maximum pressure of about 50 bar and requires a pair of pistons to multiply the pressure, the pyrotechnic gas generator produces the 300 bar injection pressure directly. Clinical trials have demonstrated that the RecoJet has excellent performance, is easy to use, and is well accepted by users. The principal disadvantage is that the reusable application device requires a relatively large one-time investment, discouraging use for short courses of medication.

Roche has adapted the RecoJet technology to single use disposable injectors that are under development, and are the subject of pending patent applications US 60/430,783 corresponding to PCT/EP 03/13290 = WO... *(No. not yet available)* and US 60/449,820 corresponding to PCT/EP 03/14729 = WO... *(No. not yet available).* The enabling discovery is that improved thermal efficiency through design optimization reduces the amount of propellant required to make an injection by about 60%. This then reduces the strength requirements of the enclosure, allowing use of disposable plastic and aluminum structures and eliminating the need for a costly reusable application device.

Initial embodiments of that amended device use electric ignition like the RecoJet. This approach requires a battery in the disposable product, which is both a cost and environmental problem. Further development has resulted in battery-free mechanically actuated ignition systems that are environmentally acceptable and low in cost.

Unlike electric ignition devices, a mechanically actuated ignition systems are generally moisture sensitive. A plastic structure is not sufficient to isolate the ignition system from the moisture in the stored medication, and barriers must be added. This increases the number of required parts, production cost and manufacturing complexity.

One of the most important parameters for pyrotechnically driven jet injection systems is the velocity of liquid medication penetrating the outer skin. Too high velocities can result in unwanted intramuscular injections, too low velocities don't assure skin penetration and cause "wet injections". The jet velocity is determined by the liquid pressure that is a function of combustion gas quantity, temperature, and gas expansion volume. Common problems with multi-part pressure housings containing parts of different materials include excessive housing volume variation caused by the sum of production tolerances of many single parts. Since quality control of many single parts is expensive, the best way to reduce the variation of housing volumes and of jet velocities would be a reduction in the number of housing parts. Such a reduction, however, cannot be achieved when combining disposable plastic and aluminum structures, as proposed in the above-mentioned patent applications.

For example, some of the impact ignition systems proposed in patent application US 60/430783 and US 60/449,820 require a hard, pressure-supporting housing, a drawn metal tube for the igniter and a softer ignition plate. The ignition plate provides a pressure-resistant seal for the drawn metal tube, and prevents leakage of combustion gases during the injection. The many parts necessary for this construction often result in excessive jet velocity variation.

US patent application US-2002/0065483-A1 discloses a "Disposable unit-dose jet injection syringe..." containing a pre-filled medication compartment formed by a deep-drawn, thin wall, metal'stamping process. While this jet injection system has a relatively low-cost body capable of withstanding high injection pressures in spite of its relatively thin wall, it has two major drawbacks:
- The nozzle diameter cannot be made much smaller than the wall thickness using a punching process, yielding a poorly focused jet stream out of the jet orifice with low skin penetration capability.
- The steel material is not a suitable container for long-term storage of liquid drugs. Therefore the inside of the deep-drawn medication chamber must be coated by a suitable polymer material, e.g. by PARYLENE®. This polymer material restricts the choice of drugs to be injected to those medications which are compatible with such polymer coatings.

It is clear from the preceding review of the NFI field and the examples of the prior art that small NFI systems have not been widely successful because of cost, complexity of use or failure to achieve high reliability. This leaves an unmet need for high quality, low cost single use disposable needle-free liquid injectors that are factory-filled with medication. Further, they must be safe to manufacture, ship, and use, and meet environmental requirements for disposal after use.

Multiple innovations are required to meet these conflicting requirements. One particular problem is to devise a structure that can safely contain the high internal pressure required for optimum injection which may be in the order of 300 bar, and yet be manufactured with high precision and at a low enough cost to be part of an economical single use disposable injector. A second particular problem is to devise a pyrotechnic power source and ignition system that has performance characteristics independent of environmental humidity, is free of environmentally regulated materials such as batteries or heavy metals, and is reliable and low in cost. A final problem is to integrate all the components and functions of a jet injector in a simple and effective product.

A further important aspect of pyrotechnically driven needle-free liquid jet injection devices is to provide a precise propellant gas expansion volume using cost-effective manufacturing processes, particularly using processes in which several components, each having a tolerance, are replaced into a single component with a smaller tolerance than the assembly of individual components.

The above mentioned problems are addressed by the invention.

The problem is solved by the invention with a device comprising the features of the enclosed independent claim 1 and an injection unit comprising the features of the enclosed independent claim 38. Further preferable features and preferred embodiments are described in the dependent claims and the following description with the attached figures.

A pyrotechnically driven needle-free liquid jet injection device for performing a needleless hypodermic injection of a liquid medication contained in the device into a body according to the invention comprises a medication unit configured and dimensioned to store a volume of liquid medication to be injected by the device, a driving unit including pyrotechnical means for generating within the device a pressure necessary for injecting the medication, said means comprising a gas generating pyrotechnical material comprised in a pressure shell surrounding at least a portion of said pyrotechnical material, and an actuation device for igniting the pyrotechnical material. Further, an injection device according to the invention has the special features of said injection device comprising a one-piece ductile pressure shell surrounding at least a portion of said pyrotechnical material, wherein the pressure shell is formed by a single deep-drawn material, preferably metal, as basic construction material for the shell and leaves at least a small opening towards the medication unit such that the hot products of combustion of said pyrotechnical material can reach the medication unit and eject the liquid medication.

The invention has recognized the opportunities for the use of low-cost deep-drawn metal components in pyrotechnically driven needle-free injectors. Broadly expressed, the liquid jet injector system according to the invention employs a ductile shell that encloses at least a portion of the pyrotechnical material.

Deep-drawing processes used to manufacture precision tubular products are an area of prior art employed in this invention. The deep-drawing process starts with a flat sheet of ductile metal. It is placed over a hole in a lower die, and a punch moves down from the top such that the portion of the flat sheet contacted by the punch is forced into the annular gap between the punch and the die. The result is that the flat sheet is formed into a cup. The process may be repeated with additional punch and die sets so that the cup is progressively deepened and refined. In some cases the parts are annealed between drawing steps to restore ductility lost to work hardening of the metal.

In deep-drawing processes length to diameter ratios of over 50 can be achieved, and complex designs with steps, flanges and changes in wall thickness can be made. Further, the process is highly automated and low in cost, and adaptable to a variety of metals including high strength stainless steel. The known deep-drawing process is described in the literature (Metals Handbook Ninth Edition, Volume 14 Forming and Forging, Joseph R. Davis, Senior Editor, ASM International, Metals Park, Ohio, pp 575-590, 767-771, (1988)).

In preferred embodiments of the invention the pyrotechnical material comprises a propellant and an igniter for igniting the propellant. In such an embodiment the ignition procedure may be simple and reliable.

Other preferred embodiments of the invention are characterized in that the actuation device or the igniter is mechanically initiated. The mechanical initiation is preferred for a reliable igninition of the pyrotechnical material which can be easily manufactured and does not need additional equipment like a power source in case of an electric ignition. The mechanical power that can be provided in a handheld injecition device for driving the actuation device or an igniter is sufficiently high.

A very favourable embodiment of the invention is characterized in that the pyrotechnical material comprises a propellant and a primer material, said primer material being so positioned with respect to said propellant that when the primer material is ignited by the igniter the hot products of combustion of said primer material ignite said propellant. In such an embodiment the ignition procedure may be simple and reliable. It is further favourable when the igniter is mechanically initiated and the primer material is an impact initiated primer material. Preferably the one-piece ductile pressure shell surrounds at least a portion of said primer material and leaves at least a small opening towards the propellant such that the hot products of combustion of said primer material can reach and ignite said propellant through said at least one small opening.

A specific favourable design of an embodiment comprising a propellant and a primer material is further characterized in that it comprises a striker for striking said primer material, said primer material being so positioned with respect to said propellant that when the striker strikes the primer material the hot products of combustion of said primer material ignite said propellant, wherein said pressure shell is designed such that an external mechanical motion of the striker causing an ignition impact on the ductile pressure shell at a location of the ductile pressure shell surrounding at least a portion of the primer material region can deform the ductile pressure shell and actuate the internal mechanically initiated primer material without penetrating the ductile pressure shell, and said deep-drawn metal of the ductile pressure shell being deformed when the striker outside of the ductile pressure shell strikes the ductile pressure shell and by means of said ductile pressure shell indenting the primer material.

In embodiments of the invention comprising a gas generating pyrotechnic propellant and a mechanically initiated ignition device the ductile shell preferably has the following attributes: A region of the ductile shell is of thickness, composition, physical properties and metallurgical condition such that a motion of an external mechanical striker can deform the shell in this region and actuate the internal mechanically initiated igniter by creating an impact force sufficiently high to ignite the igniter, e.g. a primer material within device without penetrating the shell. Such a ductile shell forms a moisture barrier that protects the igniter and propellant from external moisture or moisture diffusing from the liquid medication stored in the injector assembly.

One aspect of the invention is the discovery that pressure vessel, moisture barrier and battery-free mechanical igniter shell functions required for pyrotechnic single use jet injectors can be combined in a single low cost deep-drawn high-precision metal part. A second aspect is a uniquely simple and effective jet injector product design that incorporates such multifunctional deep-drawn metal parts.

The deep-drawn part is not a simple material substitution compared to the prior art. It combines the functions of several parts, and in the process improves function and reliability by eliminating potentially leaky joints. The key insight was that the easily deformed thin wall igniter, e.g. an igniter tube, and the thick wall propellant chambers (and in some embodiments the piston bores) could be incorporated in a single deep-drawn part that withstands operating pressures without external support.

According to a preferred additional feature the ductile pressure shell of an injection device according to the invention surrounding at least a of the pyrotechnical materarial, preferably at least a portion of the primer material, is a thin wall tube, particularly a tube with a small diameter. Preferably the diameter of that tube is smaller than the diameter of a propellant chamber including the propellant. Such a construction can be easily manufactured and is advantageous for the overall design of a practical injection device.

The medication unit, the medication cartridge (medication module) containing the medication and also the ignition means, the igniter, the actuation device and the striker for ignition of the primer material in an injection device according to the invention can be designed in any suitable manner. However, embodiments of that parts that are preferred in an injection device according to the invention are disclosed in the following documents and applications:
- EP 1 125 593 A1
- US-2002/0169412-A1
- WO 03/051432 A1
- US 60/430,783 (copy enclosed with the filing of the present European patent application; to be published as PCT/EP 03/13290 = WO... *(No*. *not yet available)*)
- US 60/449,820 (copy enclosed with the filing of the present European patent application; to be published as PCT/EP 03/14729 = WO... *(No. not yet available)*)

Concerning the above mentioned preferred embodiments and features the disclosure of these documents is incorporated by reference into the present application.

A particular preferable feature may be that the igniter comprises an ignition anvil pin within a small diameter tubular portion of the ductile pressure shell, said ignition anvil being coated with the primer material. This embodiment is a preferred design for a low-cost and effective igniter with long term stability against moisture. Further suitable igniters, e.g. hypergolic ignition, snap action (with both "1st/2nd body") and breakable rod are described in the above mentioned documents.

It may be also preferable when the ductile pressure shell is placed in a region surrounding both the primer material and the propellant. By this means the ductile pressure shell can advantageously contain the primer and the propellant.

A preferable embodiment of the invention may be characterized by a ductile pressure shell made as an open structure towards the medication unit wherein the open structure is closed towards the medication unit by a frangible foil seal, preferably a metal foil seal, that forms a moisture resistant barrier that closes the ductile pressure shell after the propellant and ignition materials are inserted, and that opens when there is significant over-pressure within the ductile pressure shell, e.g. within a propellant chamber when the propellant is ignited by the igniter. By this means protection of the primer material and the propellant against moisture can be improved.

According to an additional preferable feature of the invention the deep-drawn ductile pressure shell comprises a single deep-drawn metal part that comprises a one-side open thin wall tube in the region of the primer material and a propellant chamber in the region of the propellant, wherein the entire structure of the deep-drawn ductile pressure shell contains at least the hoop stress component of the maximum pyrotechnic pressure when the primer material and the propellant are ignited without additional structural support. Such an embodiment can be manufactured at low cost and using minimal resources, but has the advantage that it can withstand the pressure when the primer material and the propellant are ignited by the striker deforming a portion of the ductile pressure shell surrounding the primer material. For technical reasons it may be advantageous if the propellant chamber is larger or has a thicker wall than the region of the primer material.

Alternatively, another preferable embodiment can be the deep-drawn ductile pressure shell comprising a single deep-drawn metal part that comprises a one-side open thin wall tube in the region of the primer material and a larger, thick wall propellant chamber in the region of the propellant, wherein the entire structure of the deep-drawn ductile pressure shell is contained in a structural support surrounding the deep-drawn shell providing the required total structural support to contain at least the hoop stress component of the maximum pyrotechnic pressure when the primer material and the propellant are ignited. This embodiment particularly may be preferably in applications in which for some reasons, e.g. in order to minimize heat energy losses in the ignition process, the ductile pressure shell shall be very thin and/or when for security and approval reasons the injection device shall be particularly secure with respect to overpressure or burst.

Another preferable embodiment of an injection device according to the invention can be that the deep-drawn ductile pressure shell comprises a single deep-drawn metal part that comprises a one-side open thin wall tube in the region of the primer material and a (preferably larger, thick wall) propellant chamber in the region of the propellant, wherein the deep-drawn metal part forms the major part of a moisture barrier that protects the primer material and the propellant from external moisture or moisture diffusing from the liquid medication stored in the injection device.

Another preferable embodiment may be characterized in that the ductile pressure shell comprises a single deep-drawn metal part that forms a one-side open thin wall tube in the region of the primer material, and further comprises a larger, thick wall propellant chamber in the region of the propellant that is formed by a separate part.

A preferable embodiment of an igniter can be that the actuation device comprises a sufficiently strong single-part torsion spring for driving the striker onto the ductile pressure shell with sufficient energy to indent the pressure shell and ignite the primer material therein. In this embodiment it may be particularly advantageous when the torsion section of the spring is directed parallel to the symmetry axis extending from the primer material to the medication unit in order to allow for a short and slim housing and therefore for a compact device.

Another preferable embodiment may comprise the specific features that the opening of the deep-drawn ductile pressure shell is in communication with a piston or a flexible membrane such that the burning propellant exerts a force on the piston or the membrane to provide the liquid pressure necessary for the injection of the liquid medication.

A specially advantageous embodiment is achieved when the deep-drawn ductile pressure shell surrounds all of the primer material, all of the propellant, further extends towards the medication unit and surrounds at least part of a medication compartment which comprises the medication to be injected.

A preferable evolution of the injection pressure over time can be achieved if the injection device comprises a throttle vent in the form of a hole, this throttle vent being located between the pyrotechnical material, particularly the propellant, and the medication unit. The throttle vent serves to prevent excessively rapid liquid pressure rise in the medication at the beginning of the medication injection, and to provide additional pressure at a later stage of the injection.

A pyrotechnically driven needle-free liquid jet injection device according to the present invention comprises different advantages over the prior art. These advantages comprise:
- An appropriate pyrotechnic pressure source provides enough injection pressure for reliable skin penetration.
- It is possible to provide tailor-made pressure-time ejection curves by selecting an appropriate propellant or a mixture of propellants, or by optimizing the different gas expansion volumes involved.
- A precise combustion gas expansion volume is defined by a one-piece deep-drawn pressure housing which can be produced with high precision at low cost.
- The problems of gas leakage, particularly around the primer material, are solved.
- A perfect moisture seal of the primer material and of the propellant can be achieved by the deep-drawn housing surrounding them that is closed by a frangible metallic foil barrier.
- The deep-drawn housing can further be coated on its inside and/or outside by suitable materials for thermal insulation, e.g. by polymer materials. It can also additionally or alternatively be provided with a high reflective coating on its inside for the purpose of retaining the radiation emerging upon ignition of the pyrolytic material, e.g. the propellant and/or the primer material, in its interior. These measures do not reduce the number of suitable drugs that can be injected with the injection device since the part of the deep-drawn housing comprising the primer material and the propellant is not in contact with the drugs stored in the medication unit.
- The orifice (nozzle) length may be optimized independently of the steel wall thickness, since it is a separate part or is a part of a drug capsule, e.g. a glass capsule, not a hole through the steel wall.
- The device is not sensitive to the exact location where the striker hits the part of the ductile pressure shell including the primer material. This allows large production tolerances for the actuation device, e.g. the torsion spring striker
- The invention can be easily combined with a torsion spring (particularly a torsion part parallel to the symmetry axis of the deep-drawn housing) without a significant requirement of space for the spring and the actuation mechanism, thus enabling a device with a small overall volume.
- Deep-drawing is a high production rate, low cost process for manufacturing high strength components with low production tolerances.

The invention can be favorably realized both with a medication unit comprising a cylinder as the medication compartment and a piston system (these embodiments are later called piston system embodiments) and also with a medication unit comprising a compressible medication compartment (medication cartridge, medication module) (these embodiments are later called soft-shell embodiments).

In the first embodiment (piston system) the injection device comprises a piston system including at least one piston, said piston system being used for pressurizing the medication and for carrying out the injection upon ignition of the propellant, wherein the medication to be injected is contained in a medication chamber comprising a nozzle for ejection of the medication and a medication cylinder including a medication piston for pressurizing the medication for ejection through the nozzle.

An additional favorable feature of the piston system embodiment may be that the piston system comprises a second piston pushing the at least one first piston, wherein it is advantageous if the area of the first piston is smaller than the area of the second piston. Additionally it may be preferable when the piston system comprises a plunger connecting the first and the second piston.

A two piston system has the advantages that embodiments of injection devices can more easily be realized in which the medication stored in the medication unit and to be injected by the injection device has no contact with steel or stainless steel. This is important in applications in which the medication is not allowed to contact steel in order to avoid disadvantageous reactions. Another advantage of an injection device comprising at least a second piston is that aspects of leak and pressure tightness, placing of a safety relief valve and avoiding pyrotechnical material contact with the medication can be more easily taken into account.

In a piston system embodiment the deep-drawn ductile pressure shell favorably comprises a cylindrical bore containing a piston that is driven by the burning propellant to provide the mechanical power for another mechanism such as a piston or a cylinder that makes the injection.

Preferably the piston system is completely contained and moveable within the deep-drawn ductile pressure shell.

In order to further improve moisture protection the injection device may comprise a moisture barrier, preferably a metal foil, between the propellant and a piston, particularly a piston in the propellant chamber, said moisture barrier providing an additional barrier against intrusion of moisture into the propellant chamber or primer material chamber.

The injection device can also comprise a safety vent providing an opening in the deep-drawn ductile pressure shell, said safety vent being located between the medication piston and a gas pressure piston, which is driven by the propellant, and protecting the medication chamber of the medication unit from hot combustion gases leaking past an O-ring seal of a propellant chamber piston.

The injection device may also comprise a vacuum vent providing an opening in the propellant chamber and allowing evacuation of the propellant chamber upon inserting the piston or during the piston placement or before inserting the piston, further allowing a means to control the amount of humidity of the gas or air contained in the propellant chamber. The vacuum vent can be closed after the production process. It can be placed in the piston chamber wall or alternatively manufactured as thin channel in the piston and/or the plunger.

The vacuum vent can also stay open, if it is located outside the pressure tight area after the piston is inserted. It can also serve as safety vent in the case of overpressure.

A preferable piston system embodiment of the invention and preferable features of a piston system embodiment can be described as follows. The medication unit consists of a medication cylinder that contains the liquid medication and is closed by a moveable medication piston. A power piston in a power cylinder is actuated by a mechanically ignited pyrotechnic gas generator, and is mechanically connected to the medication piston such that the two pistons move in unison to make the injection. The gas generator incorporates a deep-drawn metal shell for pressure containment. This metal shell has a large diameter thick wall region that forms the power cylinder, an adjacent large diameter, thick wall region that contains the propellant being transformed into the high-pressure gas during the injection process. It is closed on one end by a small diameter, thin wall section designed to be indented by a striker on the outside to ignite impact sensitive pyrotechnic material on the inside that in turn ignites the propellant.

The striker mechanism is thereby isolated from the high pressure gas inside the metal shell. The metal shell forms the major portion of a moisture barrier. The power piston is inserted into the power cylinder after the propellant and ignition materials are put in to complete the moisture barrier. In a variation of this embodiment, the power cylinder is moulded plastic, not part of the deep-drawn metal shell. This reduces heat loss from the hot propellant. The moisture barrier is completed by a frangible metal foil seal that closes the metal shell after the propellant and ignition materials are inserted, and that opens when there is significant over-pressure (over 3 bar) within the pressure chamber. Preferably the primer material is a coating of an ignition anvil pin within a small diameter tubular portion of the deep-drawn housing.

This description of the piston system embodiment also includes complete injector configurations including the housing and ignition striker spring mechanism. Similar elements are also applicable to the second embodiment, the soft-shell embodiment, and the descriptions are not repeated.

The specific advantages of a piston system embodiment are as follows:
- The production can be performed with excellent precision at low-cost, while incorporating a a deep-drawn power piston bore in the deep-drawn part.
- Piston systems of glass or plastic materials, that are suitable for almost all liquid drugs presently known, can be used in such embodiments.
- A piston and O-Ring form an excellent seal, and an additional moisture barrier is in most cases not required.
- The amount of injected volume is precisely controlled.

In the second embodiment (soft-shell) of the injection device, the medication unit configured and dimensioned to store a volume of liquid to be injected comprises a medication module comprising a first region and a second region that are in liquid communication with each other, said first region being deformable and comprising a medication reservoir and said second region having at least one orifice, wherein the propellant chamber is configured and dimensioned such that the medication unit is located in communication or preferably at least partially within the propellant chamber and such that pressure generated within the propellant chamber would cause the first region of the medication module to deform so as to reduce the volume within the medication module for ejection of the medication.

In such an embodiment it is preferred that the deep-drawn ductile pressure shell surrounds all of the primer material, all of the propellant, further extends towards the medication unit and surrounds at least part of the medication module, leaving an opening in order to enable pressurized fluid medication to be expelled through one or more nozzles close to this opening.

In a soft-shell embodiment the deformable region of the medication module can be made of any suitable material. It is particularly advantageous when it is made of polymer material (e.g. polyethylene).

In order to achieve an improved moisture protection the injection device can comprise a rubber hat surrounding the deformable region of the medication module in order to lower the evaporation rate of water or solvent from the medication reservoir. The rubber may be silicon rubber, and can be reinforced e.g. with woven polyamide fibers, preferably polyaramide fibers. The rubber may also be a caoutchouc based material, e.g. a butyl rubber, because such materials provide a sufficiently tight diffusion barrier. The rubber hat also forms a second barrier between the hot combustion gas and the liquid medication.

A preferable soft-shell embodiment of the invention and preferable features of a soft-shell embodiment can be described as follows. A medication module, e.g. a RecoJet-type as described in US-2002/0169412-A1, is combined with a mechanically ignited pyrotechnic gas generator, and incorporates a deep-drawn metal shell for pressure containment and as the major portion of a moisture barrier. The moisture barrier is maximized by a frangible metal foil seal that closes the metal shell after the ignition materials and the propellant are inserted, and that opens when there is significant over-pressure (over 3 bar) within the pressure chamber. The metal shell has a large diameter, thick wall region that contains the propellant and the high-pressure gas during the injection process. It also has a small diameter, thin wall section that is indented by a striker on the outside to ignite impact sensitive pyrotechnic material on the inside that in turn ignites the propellant. The striker mechanism is thereby isolated from the high pressure gas inside the metal shell. In a variation of this embodiment, the metal shell is free to expand into a volume between the shell and an outer housing if the gas pressure exceeds the normal injection pressure. This expansion dissipates excess energy safely.

The specific advantages of a soft-shell embodiment are as follows:
- The materials from which the medication unit, the deformable region of the medication unit or the drug container can be manufactured are very low-cost and are suitable for drugs to be injected with the injection device.
- An improved moisture seal for the primer material or for the propellant is easily obtained by adding a frangible foil or by a rubber hat (or combination of both).
- The length of the injection device is usually shorter compared to piston embodiments.
- Hermetically sealed drug containment is achieved without sliding seals.

The invention and preferred embodiments are now described with reference to the accompanying figures. The features disclosed therein can be used singly or in combination in order to realize preferred embodiments of the invention. In the figures show:
Fig. 1 a sectional view of a first (piston type) embodiment of an injection unit according to the invention,
Fig. 2 the injector of Fig. 1 before the injection,
Fig. 3 the injector of Fig. 1 after the injection,
Fig. 4 a sectional perspective view of the injector of Fig. 1,
Fig. 5 the view of Fig. 4 in parts,
Fig. 6 an amendment of Fig. 1 including a foil as a moisture barrier,
Fig. 7 a sectional view of an amended injection device similar to the embodiment of Fig. 1,
Fig. 8 a sectional perspective view of the injector of Fig. 7,
Fig. 9 a partial view of the actuation device of Fig. 7,
Fig. 10 a view of a torsion spring and a striker for the actuation device of Fig. 7,
Fig. 11 another view of Fig. 9,
Fig. 12 another view of Fig. 10,
Fig. 13 a partially sectioned view of the uncocked actuation device of Fig. 7,
Fig. 14 the actuation device of Fig. 13 partially cocked,
Fig. 15 the actuation device of Fig. 13 fully cocked,
Fig. 16 a view of the striker of Fig. 13,
Fig. 17 a view of the striker of Fig. 14,
Fig. 18 a view of the striker of Fig. 15,
Fig. 19 a partially sectioned view of the cocked actuation device of Fig. 15,
Fig. 20 releasing the actuation device of Fig. 19,
Fig. 21 the released and uncocked actuation device of Fig. 15,
Fig. 22 a sectional view of a second (soft-shell) embodiment of an injection unit according to the invention,
Fig. 23 the injection unit of Fig. 22 with a safety volume,
Fig. 24 an amended embodiment of Fig. 22 and
Fig. 25 a variation of Fig. 1.

All of the illustrated embodiments and their variations make use of the possibility of combining a 0.1 mm wall impact igniter tube as described in the document US 60/430,783 with a thicker wall propellant container that will withstand the full actuation pressure. This simplifies the construction of the device, improves gas sealing during actuation, and provides an excellent moisture barrier to protect the ignition material and propellant. Calculations indicate that wall thickness of 0.4 mm in larger diameter (11 mm) sections, and 0.2 mm thickness in smaller diameter (5 mm) section is sufficient with 1.4301 steel in medium-hard treatment. This is a work-hardening austenitic stainless steel equivalent to US Type 304. It has high elongation before rupture, making it a good choice for containing a pressure pulse.

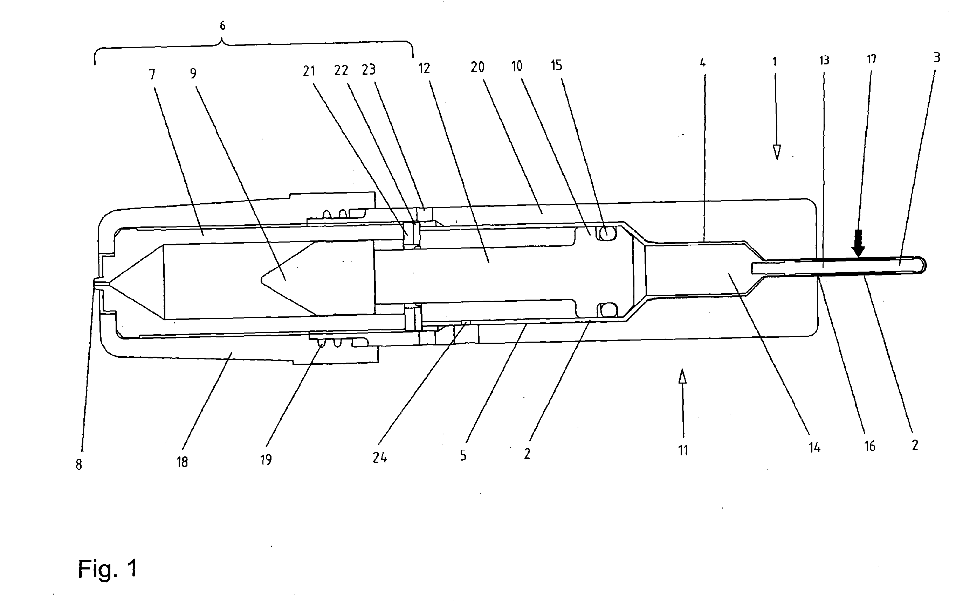

Figures 1 through 3 show a first embodiment of a jet injector (injection unit 1) incorporating a deep-drawn steel shell 2 that includes an igniter tube 3, propellant chamber 4 and power cylinder bore 5. The attached medication module 6 consists of a glass medication cylinder 7 that includes the jet nozzle 8 for ejection the medication. It contains the liquid medication and is closed by a moveable medication piston 9 made of a resilient polymer.

A power piston 10 in the power cylinder 5 is actuated by a mechanically ignited pyrotechnic gas generator 11. It is mechanically connected to the medication piston 9 by a plunger 12 that is an integral part of the power piston 10, and pushes it forward to expel the liquid medication through the jet nozzle 8.

The deep-drawn metal shell 2 provides seamless hot gas containment. It has a large diameter, thick wall region that forms the power cylinder 5. A second large diameter, thick wall region forms the propellant chamber 4. It also has a small diameter, thin wall section that is designed to be indented by a striker on the outside to ignite impact sensitive pyrotechnic primer material 13 on the inside that in turn ignites the propellant 14 contained in the propellant chamber 4. The striker mechanism is thereby isolated from the high pressure gas inside the metal shell 2. The metal shell forms the major portion of a moisture barrier for the primer material 13 and the propellant 14. The power piston plunger 12 and piston O-ring 15 isolate the propellant 14 and ignition material 13 from moisture.
They are inserted into the power cylinder 5 after the propellant 14 and ignition materials 13 are put in to complete the moisture barrier.

The mechanical igniter consists of a metal anvil pin 16 coated with impact-sensitive ignition coating. That primer material 13 preferably includes phosphorus, a strong oxidator, metal powder and binders. Many coating formulations are somewhat moisture sensitive, and require a moisture barrier to exclude external moisture and moisture diffusing from the stored medication. The coated pin 16 is coaxially positioned within the'thin wall tube 3 region of the deep-drawn metal shell 2.

When the metal tube 3 is struck on the outside by a spring-propelled striker at or near the impact point 17, it is dented and driven against the coated pin 16 to ignite the coating forming the primer material 13. The burning ignition coating generates a stream of incandescent sparks that flows out through the annulus between the thin wall tube 3 and the metal pin 16 and enters the propellant chamber 4. Here, the sparks ignite the propellant 14 and start the gas generation process. The gas pressurizes the interior of the metal shell 2, including the thin wall region around the metal pin 16, to approximately 300 bar. Even though the thin wall tube 3 has a wall thickness of only about 0.1 millimeters to facilitate high impact forces on the coated pin 16 even with relatively low striking impact, its outside diameter of about 1.6 millimeters results in pressure stresses well below the rupture stress of the steel.

The glass medication cylinder 7 is enclosed in a polymer shell 18 that screws with a thread 19 to the rear housing 20. The rear housing 20 surrounds most of the deep-drawn shell 2, and carries the axial pressure loads transmitted through the screw connection 19. The radial pressure loads are carried by the deep-drawn shell 2, and the rear housing 20 is a secondary safety barrier in case of rupture of the deep-drawn shell 2. A portion of the thin wall tube 3 region of the deep-drawn metal shell 2 extends outside the rear housing 20 where it is assessable to a spring-propelled ignition striker (not shown) striking the tube 3 at the impact point 17.

A washer-like plunger stop 21 prevents direct contact between the plunger 12 and the glass capsule 7 at the end of the injection. It also includes radial gas vents 22 that allow any gas that passes by the O-ring 15 to flow out the safety vent holes 23. The vacuum vent hole 24 allows moisture air to be evacuated from the cylinder 5 before the plunger 12 is pushed into the starting position during assembly. Without this, air is trapped and piston 12 insertion is difficult. It also vents the residual gas pressure at the end of the injection.

The same vent hole 24 or a separate safety vent 23 releases any gas that leaks past the power piston 10 to reduce the risk of gas flowing past the medication piston 9. Such flow could contaminate the medication and contact the patient. A plunger stop 21 defines the end of power piston 12 travel and prevents impact between the medication piston 9 and the end of the medication cylinder 7.

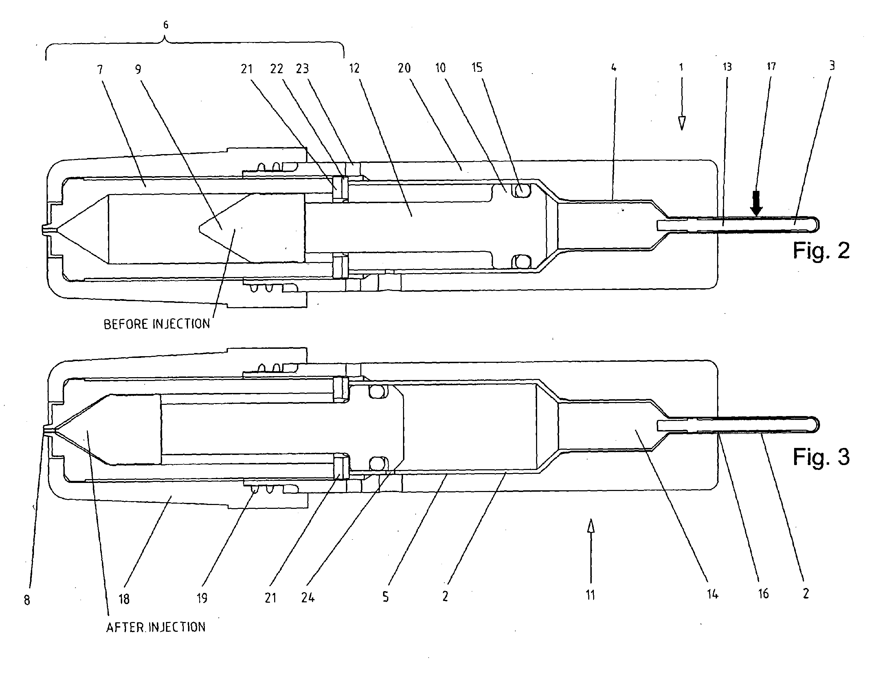

Figure 2 corresponds to Figure 1 showing the injection unit 1 before ignition of the primer material 13, Figure 3 shows the injection unit after ignition of the primer material 13 and ejection of the medication through the nozzle 8 when the power piston 10 and the medication piston 9 have reached their end points. It can be seen that the vacuum vent hole 24 is covered at the start of the injection and uncovered at the end of the injection.

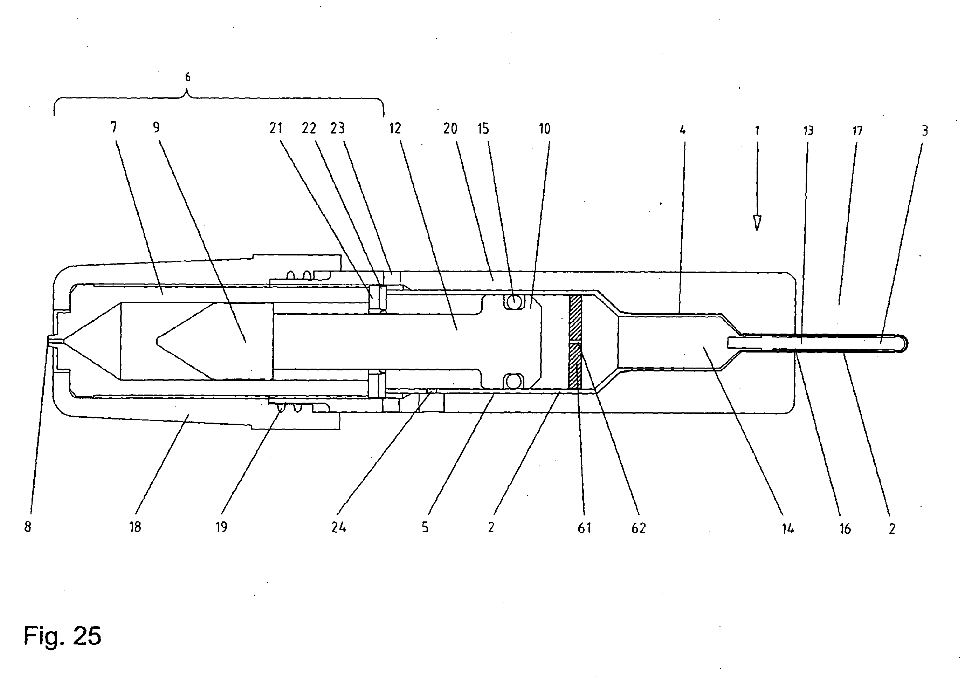

Figure 25 shows a variation of Figure 1 where a dividing wall 61 with a throttle vent 62 in the form of a hole is located between plunger 12 and propellant chamber 4. At the beginning phase of the combustion, the combustion gas pressure can only slowly propagate through the throttle vent 62. Since the liquid pressure in glass capsule 7 is proportional to the gas pressure between dividing wall 61 and plunger 12, the liquid pressure raise time can be adjusted by choosing an appropriate hole diameter for the throttle vent 62. This may be important in cases in which too fast raise times can cause glass breakage of the capsule and too slow raise times can prevent skin penetration of the jet stream and cause incomplete injections.

A second favorable effect of the throttle vent 62 is an increased injection pressure during the last phase of the injection. Too low injection pressures can prevent skin penetration of the jet stream and cause incomplete injections.

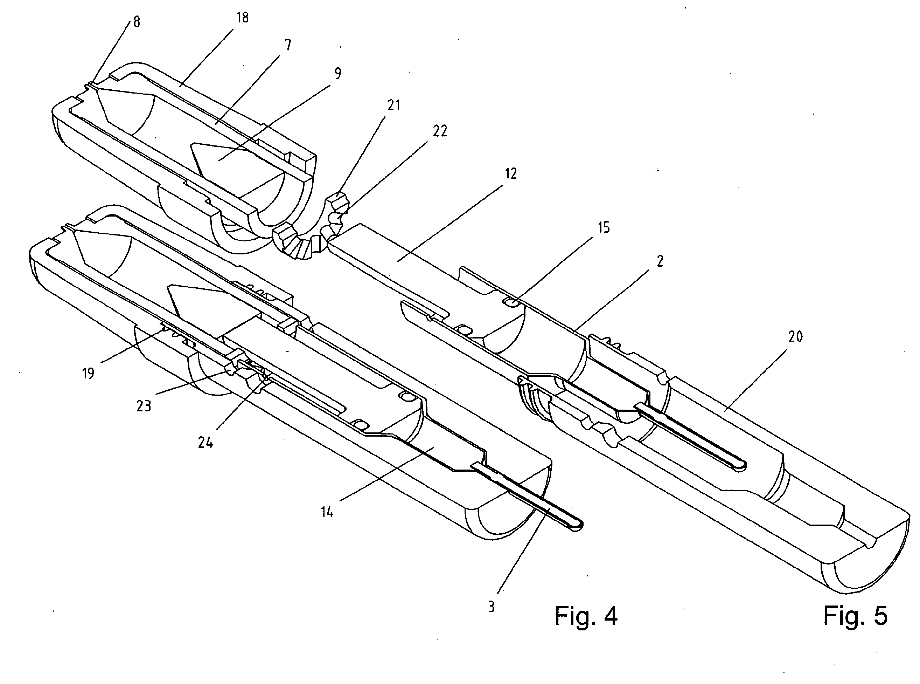

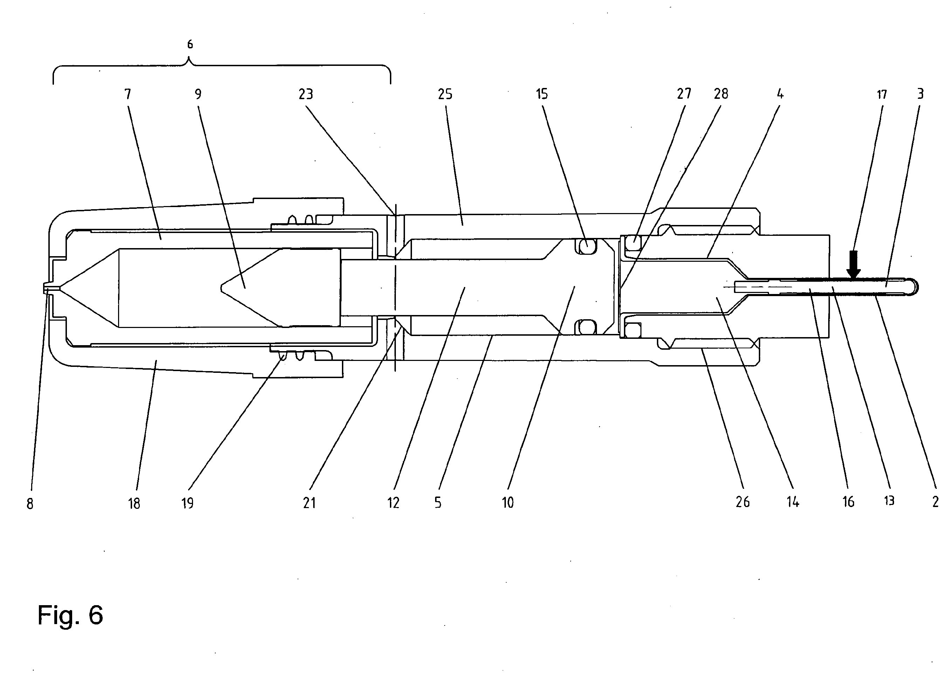

Figure 6 shows a variation in which the power cylinder bore 5 is in a plastic cylinder barrel 26, rather than part of the deep-drawn steel shell 2. The intent is to reduce the steel surface area contacted by the hot gas generated by the propellant, and the resulting heat loss. The rear of the cylinder barrel 25 is closed by a screw connection 26 and a static O-ring seal 27. A foil seal 28 forms the moisture barrier for the propellant 14 and the primer material 13. The plunger stop 21 and safety vent 23 are moulded as part of the cylinder barrel 25. A vacuum vent is not required for assembly, since the plunger 12 is inserted from the rear and the front is vented. Otherwise, this variation is similar to the embodiment illustrated in Figures 1 through 5.

It is noted that a polymer coating within a steel power cylinder bore of the first variation illustrated in Figures 1 through 5 could also reduce the heat transfer, since due to the relative long time constants involved in material heat transfer the amount of heat penetration is very small during the brief (50 to 100 millisecond) injection period. Coating of the steel inner surface of the propellant chamber 4 may also be beneficial in either variation.

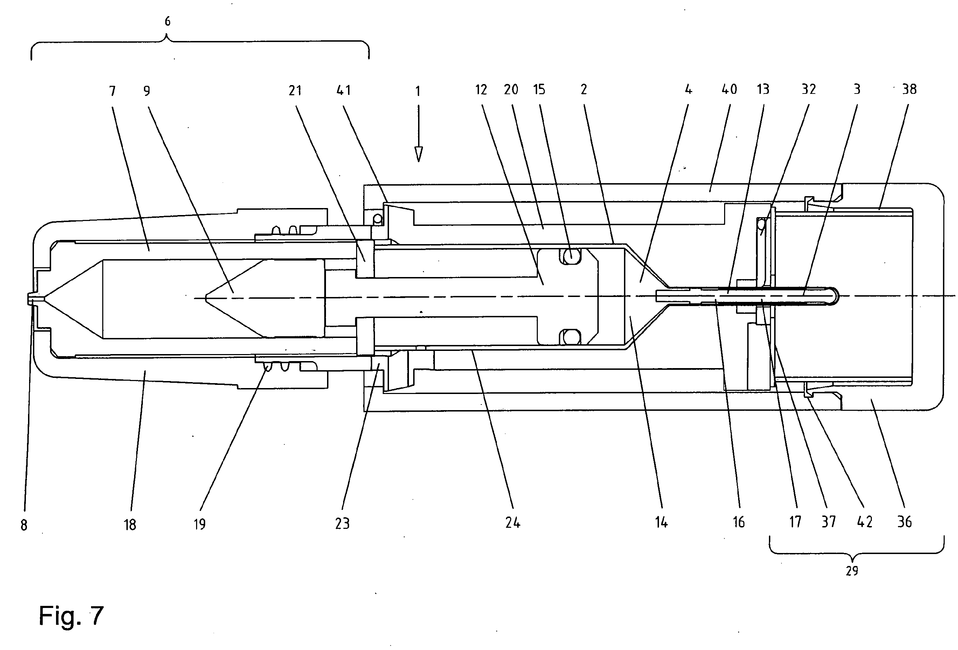

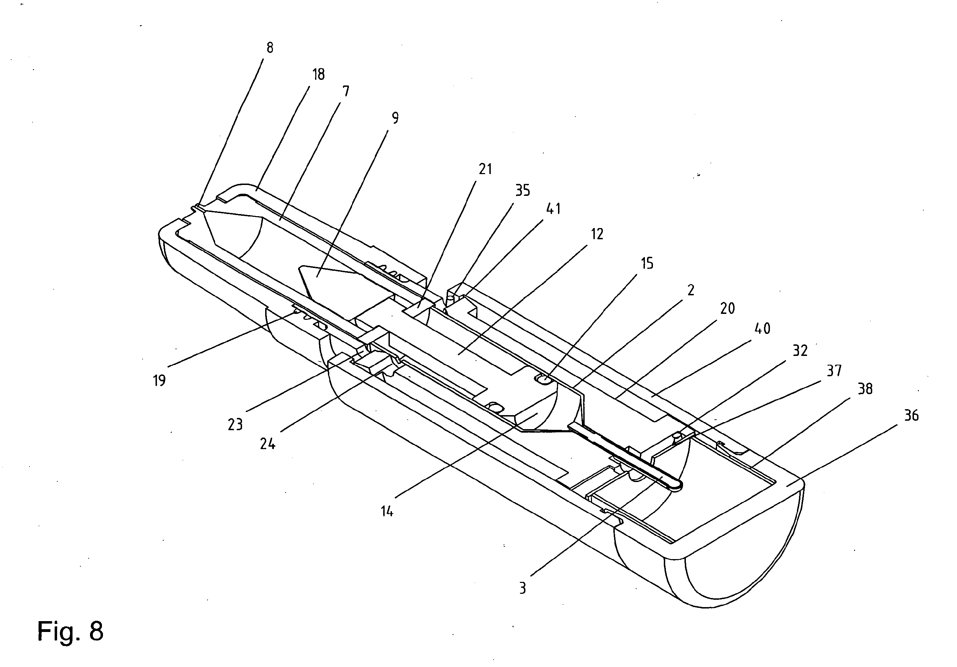

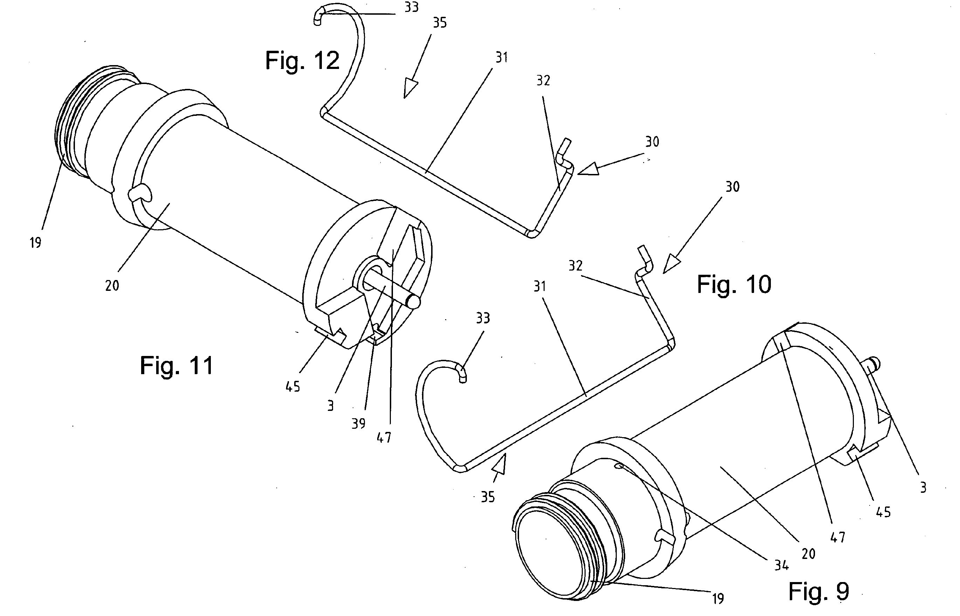

Figures 7 through 12 show a piston type injector 1 with an actuation device 29 comprising a spring striker 30 and a release mechanism for the striker 30. The active sections of the striker 30 designed as a torsion spring 35 are a torsion bar 31 and a striker arm 32 on one end of the torsion bar 31. It also includes an anchor section 33 on the opposite end of the torsion bar 31. The anchor section 33 engages an anchor hole 34 in the rear housing 20 of the injector 1 so'that it is secured against rotation.

The torsion spring 35 is supported by a spring support washer 37. A return spring 38 is also provided. The torsion bar 31 section is supported by a notch 39 in the rear housing 20 that serves as a bearing to define the center of rotation of the striker arm 32 such that the rotary path of the arm intersects the igniter tube 3 at the impact point 17. When the striker arm 32 is twisted to the cocked position, elastic energy is stored in the torsion bar 31 section. When it is suddenly released, the torsion spring rotationally accelerates the striker arm 32. The stored elastic energy is converted to kinetic energy, causing the striker arm 32 to strike and indent the igniter tube 3 and cause ignition of the primer material 13.

The mechanism to cock and release the striker spring is provided by a generally cylindrical outer housing 40 that encloses the rear housing 20, and is free to slide axially and rotate within certain limits. The rear housing 20 and injector 1 are retained in the outer housing 40 by snap connections 41, and the outer housing 40 is closed at the rear by a snap-in cap 36 with snap joint 42. A return spring 38 between the cap 36 and the rear housing 20 urges the injection out against the snap joint 42.

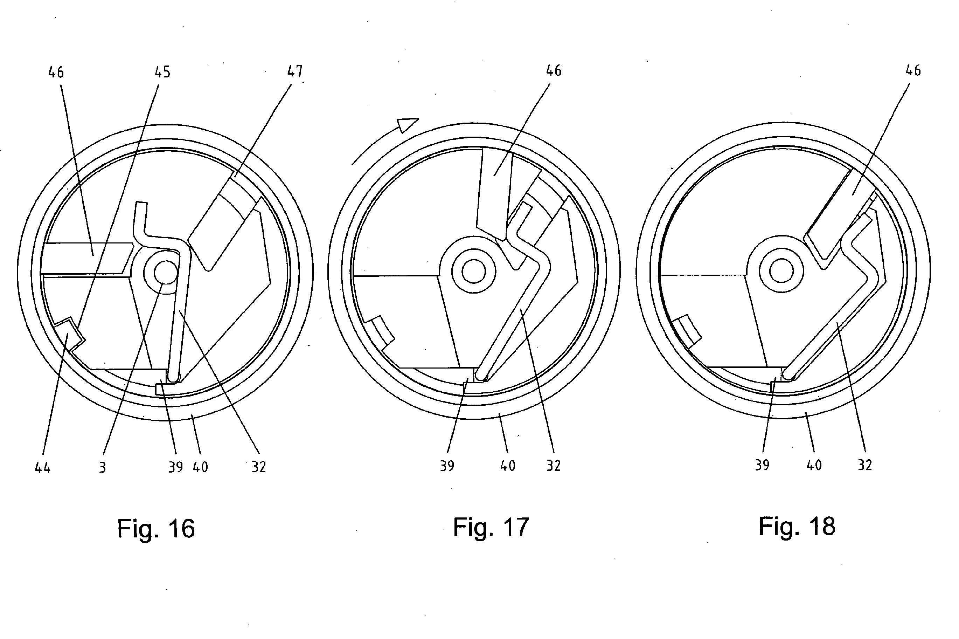

Figures 13 through 15 illustrate the injector 1 of Figure 7 in a sectional perspective view. Figures 16 through 21 show further details of this embodiment. The striker spring 35 is cocked during the factory assembly operation as shown in Figures 13 through 18. The striker spring 35 is assembled to the rear housing 20 of the injector 1 in the uncocked condition (Figures 13 and 16), and the outer sleeve 43 is slid on from the rear. The rotary alignment is determined by an index lug 44 on the inner surface of the outer housing 40 engaging a notch 45 in the rear housing 20. When the cocking lug 46, also on the inner surface of the outer housing 40, contacts the rear face of the inner housing, the index lug 44 leaves the notch 45 in the rear housing 20. This allows the outer housing 40 to rotate relative to the rear housing 20.

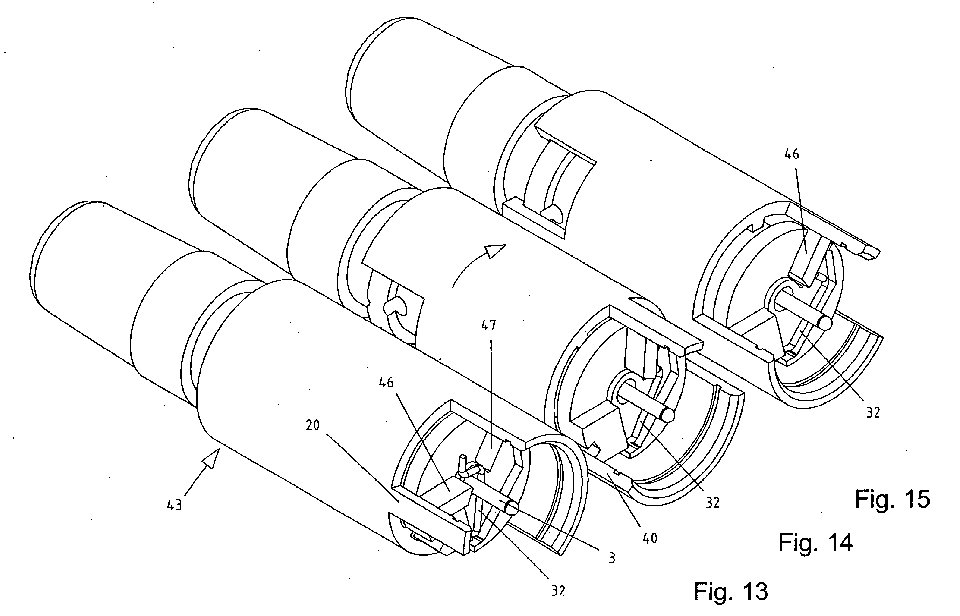

The outer housing 40 is then turned (Figures 14 and 17) as indicated by the arrow, and the cocking lug 46 rotates the striker arm 32 to the cocked position. During rotation, the index lug 44 prevents the outer housing 40 from sliding back out and prematurely releasing the striker arm 32. When the striker spring is fully cocked (Figures 15 and 18), the cocking lug 46 aligns with a cocking notch 47 in the rear housing. The outer housing 40 is then slid axially so that the cocking lug 46 engages the cocking notch 47 and locks the striker arm 32 in the cocked position. At the same time, the outer housing 40 snap connection 41 pops into place so that the outer housing 40 cannot be pulled back out. Further axial motion, which would release the striker arm 32, is prevented by a safety device (not shown). The injector assembly is completed by installing the return spring and rear cap. A washer 37 is placed between the return spring 38 and the rear housing 20 to support the spring 35 and prevent any interference between the spring 35 and the striker arm motion.

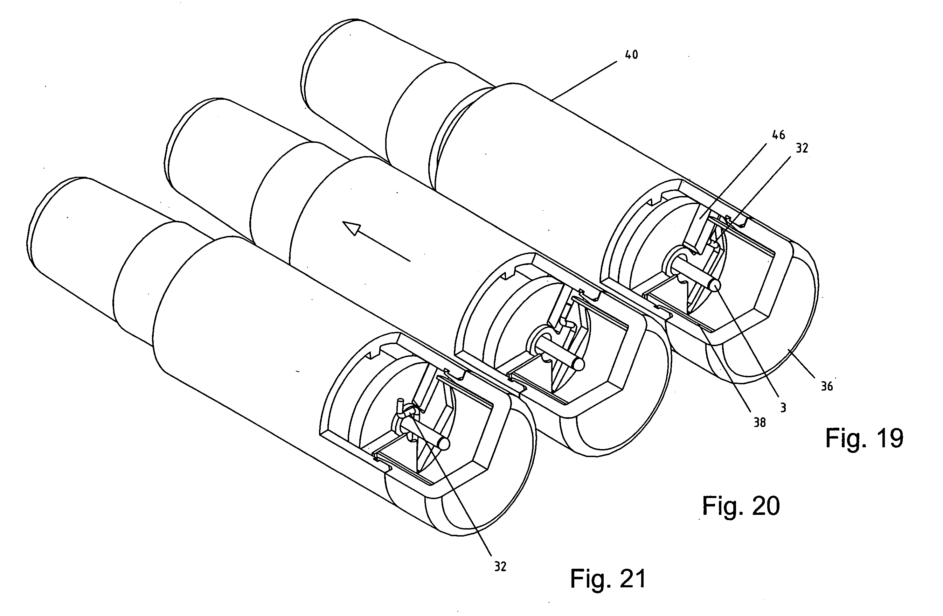

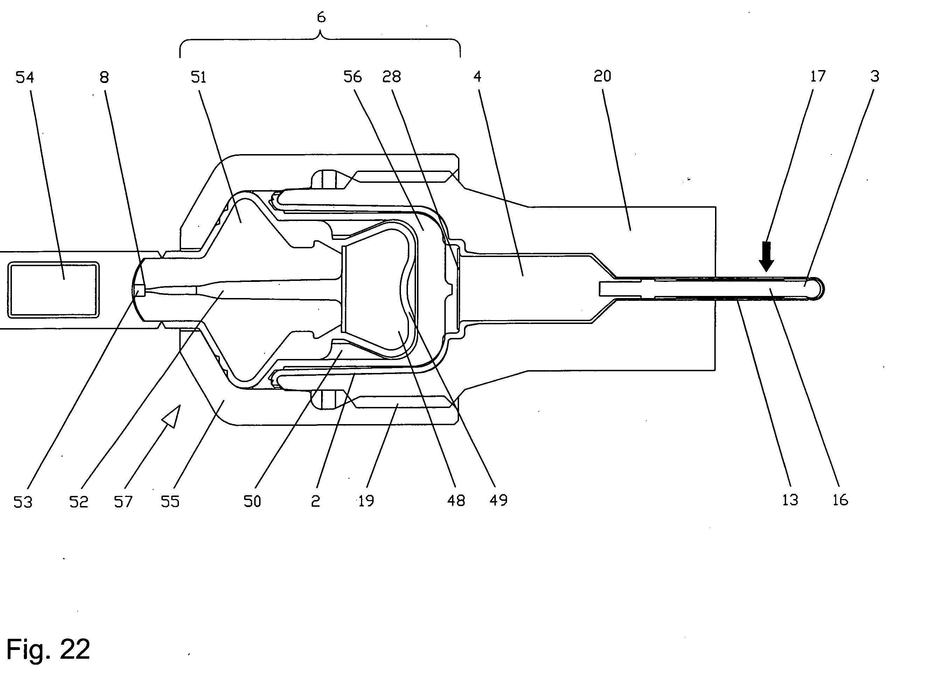

The actuation sequence is shown in Figures 19 through 21. The injection nozzle is pressed against the patient's skin by pushing into the direction indicated by the arrow and the outer housing 40 slides axially against the return spring. The cocking lug 46 slides past the striker arm 32 (Figure 20), releasing it to strike the igniter tube 3 (Figure 21) and trigger the injection.

The ignition striker cocking and release mechanisms described for the piston type embodiment embodiments illustrated in Figures 1 through 21 may be adapted to the soft-shell type embodiment illustrated in the following Figures, and the description will not be repeated.

Figure 22 shows a soft-shell embodiment of a jet injector 1 incorporating a medication module 57 of the Roche RecoJet type combined with a mechanically ignited pyrotechnic gas generator. The medication module 57 comprising a nozzle 8 and medication container 48 formed by deformable wall 49 is arranged within first chamber 50. A volume of liquid to be injected is stored in medication module 57. In preferred embodiments, the amount of this volume is in a range going from about 50 to 1000 microliters. Specific examples of this amount are e.g. 200 or 500 microliters.

Medication module 57 is a sealed medication module which comprises a nozzle body 51 and a flexible container wall 49 that hermetically encloses a portion of the nozzle 8 and forms a reservoir (medication container 48) for a liquid medication stored in sealed medication module 57. The wall 49 is deformable and collapsible.

The medication module 57 thus comprises a first region and a second region that are in liquid communication with each other. The first region is deformable and comprises the reservoir enclosed by flexible wall 49. The second region of medication module 57 comprises the nozzle 8 which has a fluid channel'52 that ends in an orifice 53 which serves as a liquid jet outlet through which liquid to be injected is ejected when an injection is performed with the injection unit 1. Medication module 57 is made of one or more suitable construction materials, e.g. polyethylene and polypropylene, which are suitable for storing medications including sensitive protein drugs.

A part of the container wall 49 forms a break-off protective cap 54 that covers the jet orifice 53 of the nozzle body 51. The cap 54 is removed by the user just prior to use of injection device 1.

The first chamber 50 is in communication with the propellant chamber 4 so that upon ignition of propellant 14 located within propellant chamber 4, gas thereby generated and exerts pressure on and deforms deformable wall 49 of the first region of medication module 57 and thereby causes ejection of the liquid medication through channel 52 and orifice 53.

The injection unit 1 illustrated in Figure 22 includes a deep-drawn metal shell 2 for pressure containment and as the major portion of a moisture barrier for the primer material 13 and the propellant 14. The moisture barrier is completed by a frangible foil seal 28 that closes the metal shell 2 after the propellant 14 and ignition materials 13 are inserted. The metal shell 2 has a large diameter, thick wall region that forms the propellant chamber 4, and a second, larger diameter, thick wall region that encloses the rear portion of the medication module 57. It also has a small diameter, thin wall section 3 that surrounds the ignition pin 16. The igniter is the same as described in the piston-type embodiment above, and the description will not be repeated.

The pressure shell 55 including the replaceable single use drug container 57 formed by the nozzle body 51 and the deformable wall 59 screws with a thread 19 to the rear housing 20. The rear housing 20 surrounds the deep-drawn shell 2, and carries the axial pressure loads transmitted through the screw connection 19. The radial pressure loads are carried by the deep-drawn shell 2, and the rear housing 20 is a secondary safety barrier. The deep-drawn shell 2 forms a seamless gas barrier to the rear. The only mechanical seal is the contact between the shell 2 and a rubber hat 56 outer lip.

A foil seal 28 isolates the propellant 14 and ignition material 13 form moisture in the rear seamless portion of the shell 2. The flexible rubber 56 hat surrounds the flexible medication container 57. Its functions include forming an additional moisture protection for propellant 14 and ignition material 13, shielding the medication container 57 from hot gas contact, and forming a second barrier between the hot gas and the medication in the event that the medication container 57 is not intact. When the propellant 14 is ignited the moisture barrier ruptures and the propellant gas pressurizes the interior of the injector structure. The pressure is transmitted through the rubber hat 56 and the flexible medication container wall 49 to the liquid medication to carry out the injection.

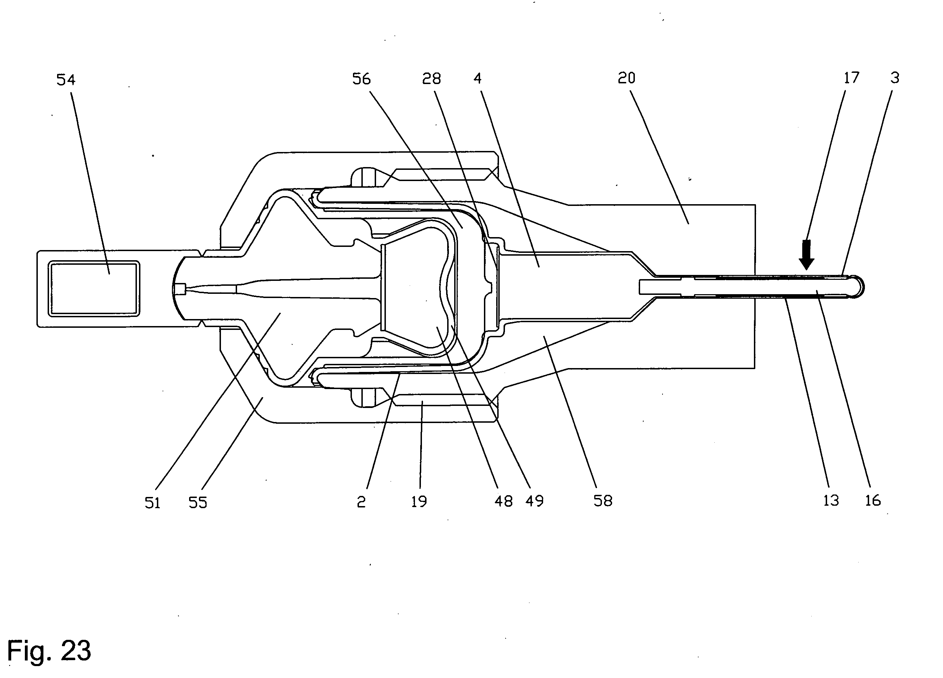

Figure 23 shows a variation of the Figure 22 design in which a safety volume 58 within the rear housing 20 surrounds the deep-drawn steel shell 2. At normal injection pressure, the steel shell 2 contains the pressure without significant distortion. As a risk minimization the safety volume 58 reduces the maximum hazard for the patient. Even in worst cases of overpressure the steel shell 2 expands into the safety volume 58 or ruptures and releases gas into the safety volume 58. In either case, the excess gas pressure is dissipated within the device.

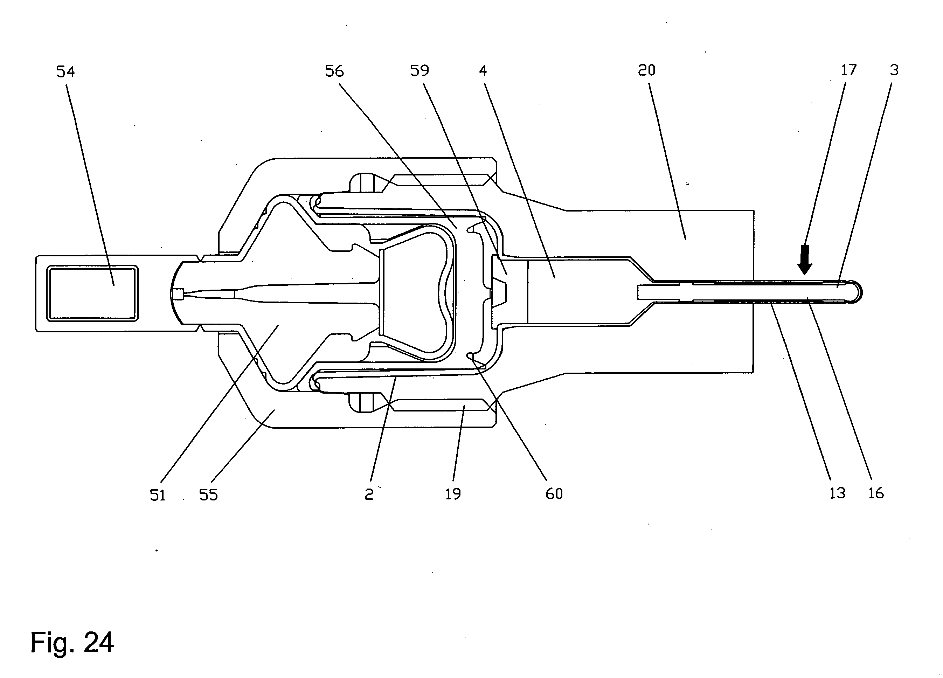

Figure 24 shows another variation of the Figure 22 design that incorporates a different moisture seal and has reduced heat loss. A rubber plug 59 replaces the foil moisture barrier 28, and a rubber hat 56 with a lip seal 60 prevents hot gas from flowing into the gap between the rubber hat 56 and the steel shell 2 and loosing heat.

### List of reference numbers

- 1: Injection unit
- 2: deep-drawn steel shell
- 3: igniter tube
- 4: propellant chamber
- 5: power cylinder bore
- 6: medication unit
- 7: medication cylinder
- 8: nozzle
- 9: medication piston
- 10: power piston
- 11: gas generator
- 12: plunger
- 13: primer material
- 14: propellant
- 15: piston O-ring
- 16: anvil pin
- 17: impact point
- 18: polymer shell
- 19: thread
- 20: rear housing
- 21: plunger stop
- 22: radial gas vent
- 23: safety vent hole
- 24: vacuum vent hole
- 25: cylinder barrel
- 26: screw connection
- 27: O-ring seal
- 28: foil seal
- 29: actuation device
- 30: striker
- 31: torsion bar
- 32: striker arm
- 33: anchor section
- 34: anchor hole
- 35: torsion spring
- 36: rear cap
- 37: spring support washer
- 38: return spring
- 39: notch
- 40: outer housing
- 41: snap connection
- 42: snap joint
- 43: outer sleeve
- 44: index lug
- 45: notch
- 46: cocking lug
- 47: cocking notch
- 48: medication container
- 49: deformable wall
- 50: first chamber
- 51: nozzle body
- 52: fluid channel
- 53: orifice
- 54: protective cap
- 55: pressure shell
- 56: rubber hat
- 57: medication module
- 58: safety volume
- 59: rubber plug
- 60: lip seal
- 61: diving wall
- 62: throttle vent

## Claims

1. Pyrotechnically driven needle-free liquid jet injection device for performing a needleless hypodermic injection of a liquid medication contained in the device into a body, said device comprising
a medication unit (6) configured and dimensioned to store a volume of liquid medication to be injected by the device,
a driving unit including pyrotechnical means for generating within the device a pressure necessary for injecting the medication, said means comprising a gas generating pyrotechnical material comprised in a pressure shell (2) surrounding at least a portion of said pyrotechnical material, and an actuation device for igniting the pyrotechnical material,
**characterized in that**
said injection device comprises a one-piece ductile pressure shell (2) surrounding at least a portion of said pyrotechnical material, wherein the pressure shell (2) is formed by a single deep-drawn material, preferably metal, as basic construction material for the shell (2) and leaves at least a small opening towards the medication unit (6) such that the hot products of combustion of said pyrotechnical material can reach the medication unit and eject the liquid medication.

2. Injection device according to claim 1, **characterized in that** the pyrotechnical material comprises a propellant (14) and an igniter for igniting the propellant (14).

3. Injection device according to claim 1 or 2, **characterized in that** the actuation device or the igniter is mechanically initiated.

4. Injection device according to claim 2 or 3, **characterized in that** the pyrotechnical material comprises a propellant (14) and a primer material (13), said primer material (13) being so positioned with respect to said propellant (14) that when the primer material (13) is ignited by the igniter the hot products of combustion of said primer material (13) ignite said propellant (14).

5. Injection device according to claim 4, **characterized in that** the igniter is mechanically initiated and the primer material (13) is an impact initiated primer material (13).

6. Injection device according to claim 4 or 5, **characterized in that** the one-piece ductile pressure shell (2) surrounds at least a portion of said primer material (13) and leaves at least a small opening towards the propellant (14) such that the hot products of combustion of said primer material (13) can reach and ignite said propellant (14) through said at least small opening.

7. Injection device according to claim 5 or 6, **characterized in that** it comprises a striker (30) for striking said primer material (13), said primer material (13) being so positioned with respect to said propellant (14) that when the striker (30) strikes the primer material (13) the hot products of combustion of said primer material (13) ignite said propellant (14), wherein said pressure shell (2) is designed such that an external mechanical motion of the striker (30) causing an ignition impact on the ductile pressure shell (2)'at a location of the ductile pressure shell (2) surrounding at least a portion of the primer material region can deform the ductile pressure shell (2) and actuate the internal mechanically initiated primer material (13) without penetrating the ductile pressure shell (2), and said deep-drawn metal of the ductile pressure shell (2) being deformed when the striker (30) outside of the ductile pressure shell (2) strikes the ductile pressure shell (2) and by means of said ductile pressure shell (2) indenting the primer material (13).

8. Injection device according to any of claims 4 to 7, **characterized in that** the ductile pressure shell (2) surrounding at least a portion of the pyrotechnical material, preferably at least a portion of the primer material (13), is a thin wall tube (3), particularly a tube (3) with a small diameter.

9. Injection device according to any of claims 4 to 8, **characterized in that** the igniter comprises an ignition anvil pin (16) within a small diameter tubular portion of the ductile pressure shell (2), said ignition anvil (16) being coated with the primer material (13).

10. Injection device according to any of claims 4 to 9, **characterized in that** the ductile pressure shell (2) is placed in a region surrounding both the primer material (16) and the propellant (14).

11. Injection device according to any of the preceding claims, **characterized in that** the ductile pressure shell (2) is made as an open structure towards the medication unit (6) and closed towards the medication unit (6) by a moisture resistant barrier formed by a frangible foil seal (28), preferably a metal foil seal, that closes the ductile pressure shell (2) after the propellant (14) and ignition materials (13) are inserted, and that opens when there is significant over-pressure within the ductile pressure shell (2).

12. Injection device according to any of claims 4 to 11, **characterized in that** the deep-drawn ductile pressure shell (2) comprises a single deep-drawn metal part that comprises a one-side open thin wall tube (3) in the region of the primer material (13) and a larger, thick wall propellant chamber (4) in the region of the propellant (14), wherein
the entire structure of the deep-drawn ductile pressure shell (2) contains without additional structural support at least the hoop stress component of the maximum pyrotechnic pressure when the primer material (13) and the propellant (14) are ignited without additional structural support.

13. Injection device according to any of claims 4 to 11, **characterized in that** the deep-drawn ductile pressure shell (2) comprises a single deep-drawn metal part that comprises a one-side open thin wall tube (3) in the region of the primer material (13) and a propellant chamber (4) in the region of the propellant (14), wherein
the entire structure of the deep-drawn ductile pressure shell (2) is contained in a structural support surrounding the deep-drawn shell providing the required total structural support to contain at least the hoop stress component of the maximum pyrotechnic pressure when the primer material and the propellant (14) are ignited.

14. Injection device according to any of claims 4 to 13, **characterized in that** the deep-drawn ductile pressure shell (2) comprises a single deep-drawn metal part that comprises a one-side open thin wall tube (3) in the region of the primer material (13) and a larger, thick wall propellant chamber (4) in the region of the propellant (14), wherein
the deep-drawn metal part forms the major part of a moisture barrier that protects the primer material (13) and the propellant (14) from external moisture or moisture diffusing from the liquid medication stored in the injection device.

15. Injection device according to any of claims 4 to 14, **characterized in that** it comprises a single deep-drawn metal part that forms a one-side open thin wall tube (3) in the region of the primer material (17), and further comprises a larger, thick wall propellant chamber (4) in the region of the propellant (14) that is formed by a separate part.

16. Injection device according to any of claims 4 to 15, **characterized in that** the actuation device comprises a sufficiently strong single-part torsion spring (35) for driving the striker (30) onto the ductile pressure shell (2)'with an energy sufficient to indent the pressure shell (2) and ignite the primer material (13) therein.

17. Injection device according to claim 16, **characterized in that** the torsion section of the spring (35) is directed parallel to the symmetry axis extending from the primer material (13) to the medication unit (6).

18. Injection device according to any of the preceding claims, **characterized in that** the opening of the deep-drawn ductile pressure shell (2) is in communication with a piston or a flexible membrane such that the burning propellant (14) exerts a force on the piston or the membrane to provide the liquid pressure necessary for the injection of the liquid medication.

19. Injection device according to any of claims 4 to 18, **characterized in that** the deep-drawn ductile pressure shell (2) surrounds all of the primer material (13), all of the propellant (14), further extends towards the medication unit (6) and surrounds at least part of a medication compartment which comprises the medication to be injected.

20. Injection device according to any of the preceding claims, **characterized in that** it comprises a throttle vent in the form of a hole, this throttle vent being located between the pyrotechnical material, particularly the propellant (14), and the medication unit and serving to prevent a too rapid initial raise time of liquid pressure in the medication upon injection of the medication and to provide additional pressure at a later stage of the injection.

21. Injection device according to any of the preceding claims, **characterized in that** pressure shell (2), particular the part of the pressure shell (2) comprising a propellant (14) and/or a primer material (13), is coated on its inside and/or outside by a thermally insulating materials.

22. Injection device according to any of the preceding claims, **characterized in that** pressure shell (2), particular the part of the pressure shell (2) comprising a propellant (14) and/or a primer material (13), is coated on its inside with a high reflective coating.

23. Injection device according to any of the preceding claims, **characterized in that** it comprises a piston system including at least one piston, said piston system being used for pressurizing the medication and for carrying out the injection upon ignition of the propellant (14), wherein the medication to be injected is contained in a medication chamber comprising a nozzle (8) for ejection of the medication and a medication cylinder (7) including a medication piston (9) for pressurizing the medication for ejection through the nozzle (8).

24. Injection device according to claim 23, **characterized in that** the piston system comprises a second piston pushing the at least one first piston.

25. Injection device according to claim 24, **characterized in that** the area of the first piston is smaller than the area of the second piston.

26. Injection device according to claim 24, **characterized in that** the piston system comprises a plunger (12) connecting the first and the second piston.

27. Injection device according to any of claims 23 to 26, **characterized in that** the deep-drawn ductile pressure shell (2) comprises a cylindrical bore containing a piston that is driven by the burning propellant (14) to provide the mechanical power for another mechanism such as a piston or a cylinder that makes the injection.

28. Injection device according to any of claims 23 to 27, **characterized in that** the piston system is completely contained and moveable within the deep-drawn ductile pressure shell (2).

29. Injection device according to any of claims 23 to 28, **characterized in that** it comprises a moisture barrier, preferably a metal foil (28), between the propellant and a piston, particularly a piston in the propellant chamber (4), said moisture barrier providing an additional barrier against intrusion of moisture into the propellant chamber (4) or primer material chamber.

30. Injection device according to any of claims 23 to 29, **characterized in that** it comprises a safety vent (23) providing an opening in the deep-drawn ductile pressure shell (2), said safety vent (23) being located between the medication piston and a gas pressure piston, which is driven'by the propellant (14), and protecting the medication chamber of the medication unit from hot combustion gases circumventing an O-ring seal (15) of a propellant chamber piston (10) in case of leakage.

31. Injection device according to any of claims 23 to 30, **characterized in that** it comprises a vacuum vent (24) providing an opening in the propellant chamber (4) and allowing evacuation of the propellant chamber (4) upon inserting the piston or before inserting the piston (10), further also allowing to control the amount of humidity of the gas or air contained in the propellant chamber (4).

32. Injection device according to any of claims 1 to 22, **characterized in that** the medication unit (6) configured and dimensioned to store a volume of liquid to be injected comprises a medication module (57) comprising a first region and a second region that are in liquid communication with each other, said first region being deformable and comprising a medication reservoir and said second region having at least one orifice (53), wherein the propellant chamber (4) is configured and dimensioned such that the medication unit (6) is located'in communication or preferably at least partially within the propellant chamber (4) and such that pressure generated within the propellant chamber (4) would cause the first region of the medication module (57) to deform so as to reduce the volume within the medication module (57) for ejection of the medication.

33. Injection device according to claim 32, **characterized in that** the deep-drawn ductile pressure shell (2) surrounds all of the primer material (13), all of the propellant (14), further extends towards the medication unit (6) and surrounds at least part of the medication module (57), leaving an opening in order to enable pressurized fluid medication to be expelled through one or more nozzles (8) close to this opening.

34. Injection device according to claim 32 or 33, **characterized in that** the deformable region of the medication module (57) is made of a polymer material.

35. Injection device according to claim 32 or 33, **characterized in that** the deformable region of the medication module is (57) made of polyethylene.

36. Injection device according to claim 32 or 33, **characterized in that** the deformable region of the medication module is a metal foil.

37. Injection device according to any of claims 32 to 36, **characterized in that** it comprises a rubber hat (56) surrounding the deformable region of the medication module (57) in order to lower the evaporation rate of water or solvent from the medication reservoir.

38. Injection unit, particularly for single use, to be used in a pyrotechnically driven needle-free liquid jet injection device according to any of the preceding claims for performing a needleless hypodermic injection of a liquid medication contained in the injection unit into a body, said injection unit comprising
a medication unit (6) configured and dimensioned to store a volume of liquid medication to be injected by the unit,
pyrotechnical means for generating within the injection unit a pressure necessary for injecting the medication, said means comprising a gas generating pyrotechnical material comprised in a pressure shell (2) surrounding at least a portion of said pyrotechnical material contained in said unit, and an actuation device for igniting the pyrotechnical material,
**characterized in that**
said injection unit comprises a one-piece ductile pressure shell (2) surrounding at least a portion of said pyrotechnical material, said pressure shell (2) being formed by a single deep-drawn material, preferably metal, as basic construction material for the shell (2) and leaving at least a small opening towards the propellant (14) such that the hot products of combustion of said pyrotechnical material can reach the medication unit and eject the liquid medication.

39. Injection unit according to claim 38, **characterized in that** it comprises at least one more of the features of any of claims 1 to 37.
